(19) Europäisches Patentamt
European Patent Office
Office européen des brevets

(11) Veröffentlichungsnummer: **0 108 709**
**A2**

(12) # EUROPÄISCHE PATENTANMELDUNG

(21) Anmeldenummer: 83810447.9

(22) Anmeldetag: 03.10.83

(51) Int. Cl.³: C 07 D 211/46
C 07 D 211/16, C 07 D 498/10
C 07 D 471/10, C 07 D 491/10
C 08 K 5/34, C 08 K 5/35
//(C07D498/10, 263/00, 221/00),
(C07D491/10, 319/00, 221/00),
(C07D471/10, 235/00, 221/00)

(30) Priorität: 08.10.82 CH 5924/82

(43) Veröffentlichungstag der Anmeldung:
16.05.84 Patentblatt 84/20

(84) Benannte Vertragsstaaten:
DE FR GB IT

(71) Anmelder: CIBA-GEIGY AG
Postfach
CH-4002 Basel(CH)

(72) Erfinder: Karrer, Friedrich, Dr.
Rebbergstrasse 5
CH-4800 Zofingen(CH)

(54) **Neue Polyalkylpiperidinverbindungen.**

(57) Durch Umsetzung von Verbindungen der Formel

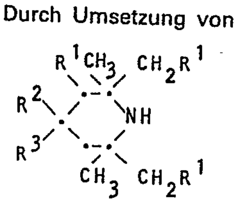

mit Di- oder Triisocyanaten der Formel $R^4(NCO)_n$ bei niedriger Temperatur erhält man die entsprechenden 1-Carbamoylverbindungen der Formel I

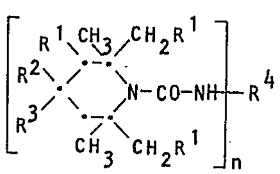

I

worin n 2 oder 3 ist und $R^1$, $R^2$, $R^3$ und $R^4$ die im Anspruch 1 definierte Bedeutung haben. Die Verbindungen der Formel I sind Lichtschutzmittel für organische Materialien, insbesondere für Lacke.

CIBA-GEIGY AG
Basel (Schweiz)

3-14137/+

## Neue Polyalkylpiperidinverbindungen

Die vorliegende Erfindung betrifft neue Polyalkylpiperidinverbindungen ein Verfahren zu ihrer Herstellung und mit diesen Verbindungen stabilisierte Kunststoffe.

In 2- und 6-Stellung alkylierte Piperidine gehören zur Klasse der sterisch gehinderten Amine, die als Stabilisatoren für lichtempfindliche organische Materialien verwendbar sind. Solche Piperidinverbindungen besitzen meist in 4-Stellung eine polare Gruppe und können in 1-Stellung unsubstituiert oder durch einwertige Reste substituiert sein. In der DE-OS 2 338 076 sind auch Bis-piperidinverbindungen beschrieben, in denen zwei in 4-Stellung substituierte 2,2,6,6-Tetramethylpiperidine durch 2-wertige Reste in 1-Stellung miteinander verknüpft sind. Die dort verwendeten 2-wertigen Reste sind insbesondere Alkylenreste und durch Ether-, Ester- oder Thioethergruppen unterbrochene Alkylenreste sowie aliphatische Dicarbonsäurereste.

Solche Bis-piperidinderivate haben gegenüber Monopiperidinderivaten den allgemeinen Vorteil der geringeren Flüchtigkeit und Extrahierbarkeit. Es wäre daher auch von Interesse, eine solche Molekülvergrösserung durch Reaktion von in 1-Stellung unsubstituierten Polyalkylpiperidinen mit Diisocyanaten (oder Triisocyanaten) zu erreichen. Es ist jedoch bekannt, dass die sterisch gehinderten Piperidine einer Carbamoylierung in 1-Stellung nur sehr schwer zugänglich sind. So wird in Beispiel 87 der DE-OS 2 258 752 bei der Umsetzung von 2,2,6,6-Tetramethylpiperidinol-4 mit überschüssigem Methylisocyanat nach 24 Stunden Reaktion in siedendem Benzol nur ein 80 %ig reines Produkt erhalten, während gemäss Beispiel 45

derselben Publikation die Umsetzung von 1-substituierten Tetramethyl-4-piperidinolen mit Methylisocyanat ohne Schwierigkeiten verläuft. Man konnte daraus schliessen, dass die Carbamoylierung des sterisch gehinderten Piperidin-Stickstoffes nur sehr langsam und unvollständig verläuft, so dass keine Chancen bestanden, durch Umsetzung mit Diisocyanaten zu reinen Bis-piperidinverbindungen zu gelangen.

In der DE-OS 2 834 455 wird die Umsetzung von 4-Spirooxazolidonpolyalkylpiperidinen mit Diisocyanaten beschrieben, wobei eine Reaktion nur am Oxazolidon-Stickstoff, nicht am Piperidin-Stickstoff stattfindet:

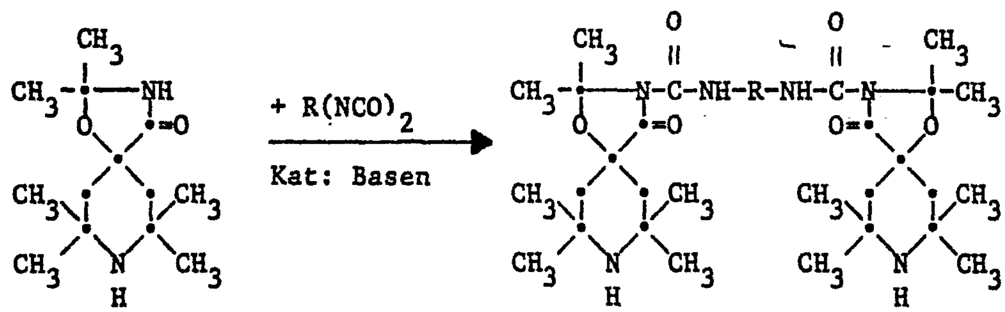


Unter den dort beschriebenen Reaktionsbedingungen (15 Stunden in siedendem Toluol) wird also keine merkliche Menge an 1-Carbamoylprodukten erhalten.

Es war daher überraschend zu finden, dass bei der Umsetzung von 1-unsubstituierten Polyalkylpiperidinderivaten mit Di- oder Triisocyanaten bei Raumtemperatur innerhalb kurzer Zeit sich in praktisch quantitativer Menge die entsprechenden Bis- bzw. Tris-1-carbamoylpiperidine bilden. Wider aller Erwartung verläuft also die 1-Carbamoylierung, die in der Wärme nicht oder nur unvollständig verläuft, in der Kälte rasch und in hoher Ausbeute. Durch dieses Herstellungsverfahren sind solche Bis- und Tris-1-carbamoylpiperidine erstmals zugänglich geworden.

Ein Gegenstand der vorliegenden Erfindung sind daher Verbindungen der Formel I,

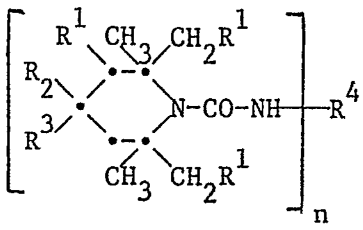 I

worin n 2 oder 3 ist,

$R^1$ Wasserstoff oder $C_1$-$C_4$-Alkyl ist,

$R^2$ Wasserstoff, -OH, $-OR^5$, $-COOR^6$, $-CH_2COOR^6$, $-CH_2CN$, -CN, $-N(R^7)_2$, $-CO-N(R^{7a})_2$, $-CH_2CH_2N(R^7)_2$, $-N(R^{7a})-CO-R^8$, $--O-CO-R^9$, $-OCH_2CH_2-CN$, $-N(R^{7a})-CO-O-R^{10}$, $-O-CO-O-R^{10}$, $-O-CO-N(R^{7a})_2$ oder eine Gruppe

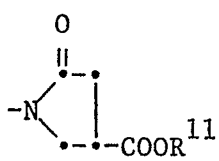 , 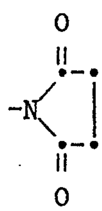 , 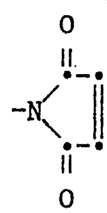 , 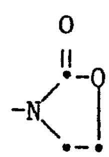

oder 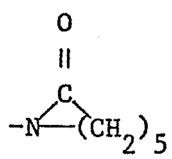 bedeutet,

$R^3$ Wasserstoff ist oder $R^2$ und $R^3$ zusammen O=, NC-CH= oder eine Gruppe der Formel II, III, IV, V oder VI bedeuten,

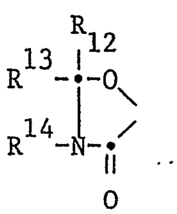 , 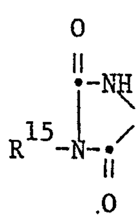 , 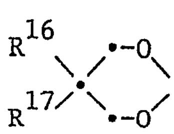 ,

II III IV

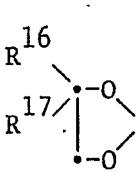 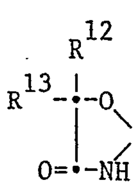

V VI

$R^4$ ein n-wertiger Rest eines aliphatischen, cycloaliphatischen, aromatischen oder aromatisch-aliphatischen Di- oder Triisocyanates ist,

$R^5$ $C_1$-$C_{12}$-Alkyl, $C_3$-$C_{14}$-Oxaalkyl, $C_3$-$C_5$-Alkenyl, Propargyl, Benzyl, oder $-(CH_2CH_2O)_p$-H mit p = 1-10 bedeutet,

$R^6$ Wasserstoff, $C_1$-$C_{18}$-Alkyl, $C_3$-$C_5$-Alkenyl, $C_5$-$C_8$-Cycloalkyl, $C_6$-$C_{10}$-Aryl, $C_7$-$C_{12}$-Alkylaryl, $C_7$-$C_{12}$-Phenylalkyl oder eine Gruppe der Formel VI bedeutet,

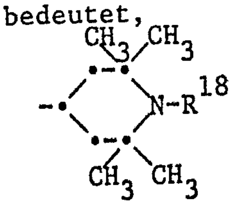

VI

$R^7$ $C_1$-$C_{18}$-Alkyl, $C_2$-$C_{14}$-Oxaalkyl, $C_3$-$C_5$-Alkenyl, $C_5$-$C_8$-Cycloalkyl, $C_7$-$C_{12}$-Phenylalkyl, $C_6$-$C_{10}$-Aryl, $C_2$-$C_4$-Hydroxyalkyl oder $C_7$-$C_{10}$-Cycloalkyl-alkyl bedeutet, oder beide Reste $R^7$ zusammen mit dem N-Atom, an das sie gebunden sind, einen 5-7-gliedrigen Ring bilden,

$R^{7a}$ Wasserstoff ist oder eine der für $R^7$ gegebenen Bedeutungen hat,

$R^8$ $C_1$-$C_{12}$-Alkyl, $C_3$-$C_{14}$-Oxaalkyl, durch -COOH oder -COO($C_1$-$C_4$-Alkyl) substituiertes $C_2$-$C_{14}$-Alkyl, $C_2$-$C_5$-Alkenyl, $C_5$-$C_8$-Cycloalkyl, $C_7$-$C_{12}$-Phenylalkyl, $C_6$-$C_{10}$-Aryl oder $C_7$-$C_{12}$-Alkylaryl bedeutet,

$R^9$ $C_1$-$C_{18}$-Alkyl, $C_3$-$C_{14}$-Oxaalkyl, durch -COOH oder -COO($C_1$-$C_4$-Alkyl) substituiertes $C_2$-$C_{14}$-Alkyl, $C_2$-$C_5$-Alkenyl, $C_5$-$C_8$-Cycloalkyl, $C_6$-$C_{10}$-Aryl, $C_7$-$C_{12}$-Phenylalkyl, $C_7$-$C_{12}$-Alkylaryl oder durch OH und 1 - 3 $C_1$-$C_4$-Alkylgruppen substituiertes Phenyl oder $C_7$-$C_{10}$ Phenylalkyl bedeutet,

$R^{10}$ $C_1$-$C_{12}$-Alkyl, $C_3$-$C_5$-Alkenyl, $C_5$-$C_8$-Cycloalkyl, Phenyl oder $C_7$-$C_{12}$-Phenylalkyl bedeutet,

$R^{11}$ Wasserstoff oder $C_1$-$C_8$-Alkyl ist,

$R^{12}$ Wasserstoff, $C_1$-$C_{12}$-Alkyl, $C_5$-$C_8$-Cycloalkyl oder Benzyl und

$R^{13}$ $C_1$-$C_{12}$-Alkyl, $C_5$-$C_8$-Cycloalkyl oder Phenyl ist oder

$R^{12}$ und $R^{13}$ zusammen mit dem C-Atom, an das sie gebunden sind, einen $C_6$-$C_{14}$-Cycloalkan- oder Alkylcycloalkanring bilden,

$R^{14}$ Wasserstoff, $C_1$-$C_{12}$-Alkyl, $C_3$-$C_5$-Alkenyl, $C_5$-$C_8$-Cycloalkyl, $C_2$-$C_4$-Hydroxyalkyl, $C_2$-$C_{10}$-Alkanoyl, Benzoyl, $C_7$-$C_{12}$-Phenylalkyl oder eine Gruppe R-NH-CO- ist, worin R $C_1$-$C_8$-Alkyl oder Phenyl bedeutet,

$R^{15}$ Wasserstoff, $C_1$-$C_{18}$-Alkyl, $C_3$-$C_5$-Alkenyl, $C_2$-$C_4$-Hydroxyalkyl, $C_5$-$C_8$-Cycloalkyl, $C_7$-$C_{12}$-Phenylalkyl oder Glycidyl bedeutet,

$R^{16}$ Wasserstoff oder $C_1$-$C_4$-Alkyl ist,

$R^{17}$ Wasserstoff, Methyl, Ethyl oder Hydroxymethyl bedeutet und

$R^{18}$ Wasserstoff, $C_1$-$C_4$-Alkyl, Allyl, Benzyl, Acetyl oder Acryloyl bedeutet.

In Formel I können die Substituenten $R^1$, $R^5$, $R^6$, $R^7$, $R^8$, $R^9$, $R^{10}$, $R^{11}$, $R^{12}$, $R^{13}$, $R^{14}$, $R^{15}$, $R^{16}$ und $R^{18}$ Alkyl sein und sie können dabei einen unverzweigten oder verzweigten Alkylrest darstellen. Beispiele für solche Alkylreste - innerhalb der definierten C-Anzahl - sind Methyl, Ethyl, n-Propyl, Isopropyl, sec.-Butyl, n-Butyl, tert.-Butyl, Isoamyl, n-Hexyl, 2-Ethylbutyl, Isoheptyl, n-Octyl, 2-Ethylhexyl, n-Nonyl, Isodecyl, 3,3,5,5-Tetramethylhexyl, n-Dodecyl, n-Tetradecyl, n-Hexadecyl, n-Octadecyl.

$R^7$, $R^{14}$ und $R^{15}$ als Hydroxyalkyl können z.B. 2-Hydroxyethyl, 2-Hydroxypropyl, 3-Hydroxypropyl oder 2-Hydroxybutyl sein. $R^5$, $R^6$, $R^8$ und $R^9$ als Oxaalkyl können Mono- oder Polyoxaalkyl bedeuten und können z.B. 2-Ethoxyethyl, 2-Isopropoxyethyl, Butoxymethyl, 2-Hexyloxyethyl, 3,6-Dioxaheptyl, 3,6-Dioxadecyl, 3,6,9-Trioxadecyl oder 3-Dodecyloxypropyl sein. $R^7$ als Oxaalkyl kann darüber hinaus auch Methoxymethyl sein.

$R^8$ und $R^9$ als durch -COOH oder -COO($C_1$-$C_4$-Alkyl) substituiertes $C_2$-$C_{14}$-Alkyl können z.B. 2-Carboxyethyl, 2-Methoxycarbonyl-butyl, 2-Ethoxycarbonyl-decyl, 2-Carboxy-tetradecyl oder 2-Isopropoxycarbonyl-tetradecyl sein.

$R^5$, $R^7$, $R^{10}$, $R^{14}$, $R^{15}$ als Alkenyl können z.B. Methallyl, 2-Butenyl oder 2-Methyl-2-butenyl, vorzugsweise jedoch Allyl sein. $R^8$ und $R^9$ als Alkenyl können auch Vinyl, 1-Methylvinyl oder 2-Methylvinyl sein.

$R^6$, $R^7$, $R^8$, $R^9$, $R^{10}$, $R^{12}$, $R^{13}$, $R^{14}$ und $R^{15}$ können Cycloalkyl sein wie z.B. Cyclopentyl, Cyclohexyl, Cycloheptyl oder Cyclooctyl, $R^7$ als Cycloalkyl-alkyl kann z.B. Cyclohexylmethyl, Cyclopentylethyl oder 3-Cyclohexylpropyl sein.

$R^6$, $R^7$, $R^8$ und $R^9$ als Aryl können Phenyl oder Naphthyl sein. $R^9$ als durch OH und Alkyl substituiertes Phenyl oder Phenylalkyl kann z.B. 4-Hydroxy-3,5-di-tert.-butylphenyl, 4-Hydroxy-3-methyl-5-tert.-butylbenzyl, 3-Hydroxy-4-tert.-butylbenzyl, 2-(4-Hydroxy-3,5-di-tert.-butylphenyl)-ethyl oder -propyl sein.

$R^6$, $R^8$ und $R^7$ als $C_7$-$C_{12}$-Alkylaryl können z.B. Tolyl, Xylyl, 4-tert.-Butylphenyl, 4-Methyl-1-naphthyl oder 3-Ethylphenyl sein. $R^6$, $R^7$, $R^8$, $R^9$, $R^{10}$, $R^{14}$ und $R^{15}$ als Phenylalkyl können z.B. Benzyl, 2-Phenylethyl, 2-Phenylpropyl, 3-Phenylpropyl, 4-Phenylbutyl oder 4-Methylbenzyl sein.

$R^{14}$ als $C_2$-$C_{10}$-Alkanoyl kann z.B. Acetyl, Propionyl, Butyryl, Hexanoyl, $\alpha,\alpha$-Dimethylpropionyl, Octanoyl oder Decanoyl sein. Wenn die Gruppe $N(R^7)_2$ einen 5-7-gliedrigen Ring darstellt, so kann dieser z.B. ein Pyrrolidin-, Piperidin-, Morpholin- oder 4-Alkylpiperazinring sein.

Wenn $R^{12}$ und $R^{13}$ zusammen mit dem bindenden C-Atom einen Cycloalkan- oder Alkylcycloalkanring bilden, so kann dies z.B. ein Cyclopentan-, Cyclohexan-, Mono- oder Dimethylcyclohexan-, Ethylcyclopentan-, Cyclooctan- oder Cyclododecanring sein.

$R^4$ als zweiwertiger Rest eines aliphatischen Diisocyanates kann z.B. Di-, Tri-, Tetra-, Hexa-, Octa-, Deca- oder Dodecamethylen

sein. $R^4$ als Rest eines cycloaliphatischen Diisocyanates kann z.B. 1,4-Cyclohexylen, 4-Methyl-1,3-cyclohexylen, der Rest

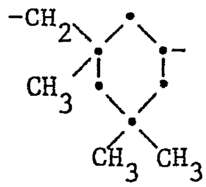

oder ein Rest der Formel

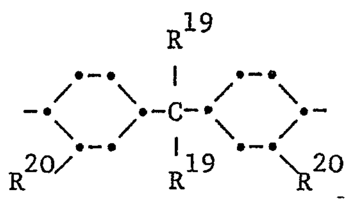 sein,

worin $R^{19}$ H, $CH_3$ oder $C_2H_5$ und $R^{20}$ H oder $CH_3$ ist. $R^4$ als Rest eines aromatischen oder aromatisch-aliphatischen Diisocyanates kann z.B. 1,4-Phenylen, 1,3-Phenylen, 2,4-Tolylen, 2,6-Tolylen, 1,4-Naphthylen, 1,5-Naphthylen, 4,4'-Diphenylen oder ein Rest der Formel

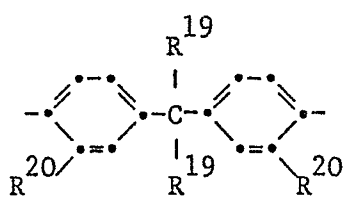 sein.

$R^4$ als dreiwertiger Rest eines Triisocyanates kann aliphatisch, cycloaliphatisch oder aromatisch-aliphatisch sein. Beispiele hierfür sind die Reste der Formeln

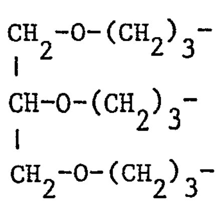 ,

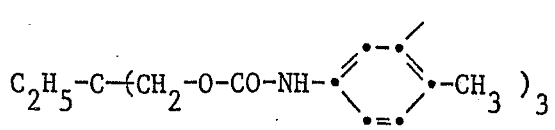 ,

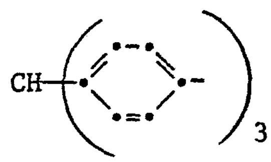 oder 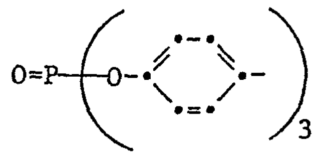 .

Bevorzugt ist $R^4$ bei n = 2 ein zweiwertiger gesättigter aliphatischer Rest mit 2 - 16 C-Atomen, ein zweiwertiger cycloaliphatischer Rest mit 5 - 20 C-Atomen oder ein zweiwertiger aromatischer oder aromatisch-aliphatischer Rest mit 6 - 20 C-Atomen, und bei n = 3 ein dreiwertiger aromatisch-aliphatischer Rest mit 10-30 C-Atomen.

Unter den Verbindungen der Formel I sind solche bevorzugt, worin $R^1$ Wasserstoff sind, diese stellen Derivate des 2,2,6,6-Tetramethylpiperidin dar.

Bevorzugte Verbindungen der Formel I sind weiterhin solche, worin $R^1$ Wasserstoff, $R^2$ -OH, $-OR^5$, $-CH_2COOR^6$, $-CH_2CN$, $-N(R^7)-CO-R^8$, $-O-CO-R^9$, $-N(R^7)-CO-OR^{10}$ oder $-O-CO-N(R^7)_2$ bedeutet, $R^3$ Wasserstoff ist oder $R^2$ und $R^3$ zusammen O= oder eine Gruppe der Formeln II, III, IV oder V bedeuten, $R^4$ bei n = 2 $C_2$-$C_{12}$-Alkylen, Phenylen, Naphthylen, Tolylen oder eine Gruppe der Formeln

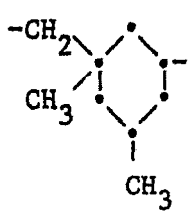 , 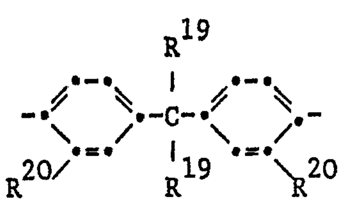 oder

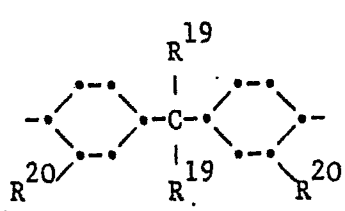

bedeutet, worin $R^{19}$ Wasserstoff, Methyl oder Ethyl ist und $R^{20}$ Wasserstoff oder Methyl ist,
und bei n = 3 eine Gruppe der Formel

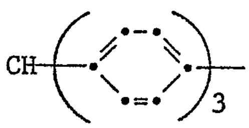 oder 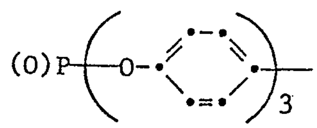 bedeutet,

$R^5$ $C_1$-$C_{12}$-Alkyl, $C_3$-$C_5$-Alkenyl oder Benzyl ist, $R^6$ $C_1$-$C_4$-Alkyl oder Cyclohexyl ist, $R^7$ $C_1$-$C_{12}$-Alkyl, Cyclohexyl, Allyl oder Phenyl ist, $R^8$ und $R^9$ $C_1$-$C_{12}$-Alkyl, $C_2$-$C_3$-Alkenyl, Phenyl, Benzyl oder Cyclohexyl bedeuten und $R^{10}$ $C_1$-$C_{12}$-Alkyl oder Phenyl ist.

Beispiele für einzelne Verbindungen der Formel I sind die Verbindungen der folgenden Formeln:

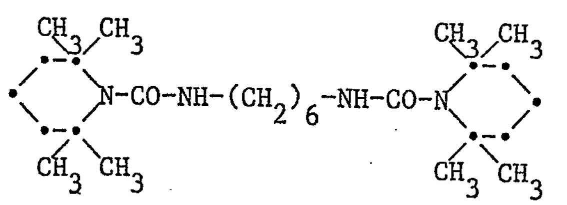

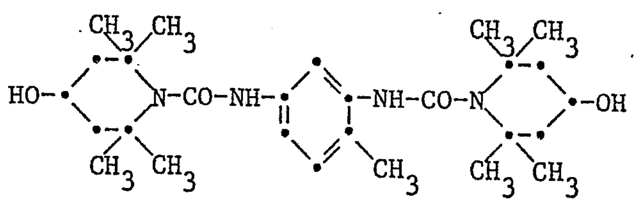

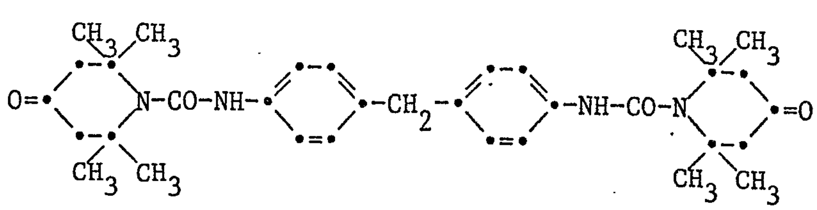

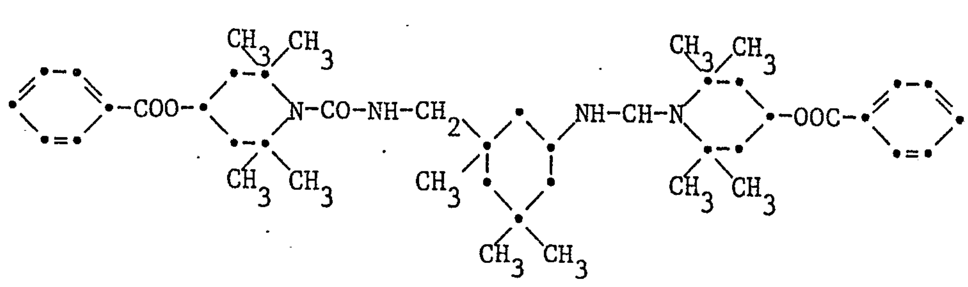

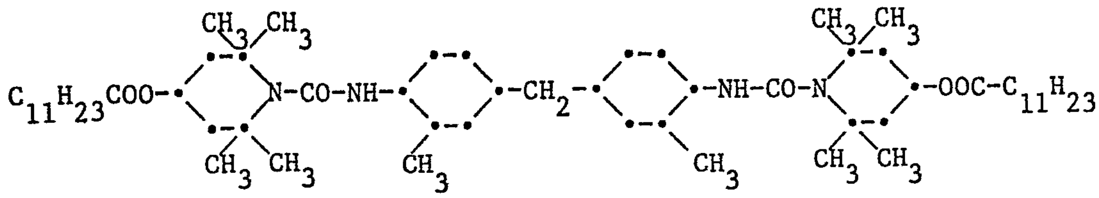

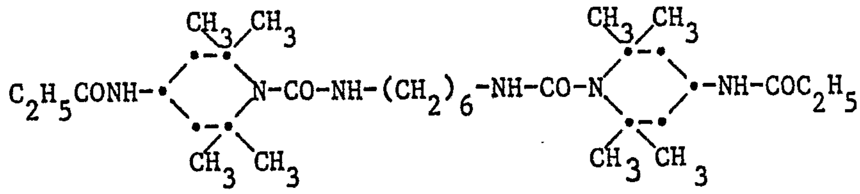

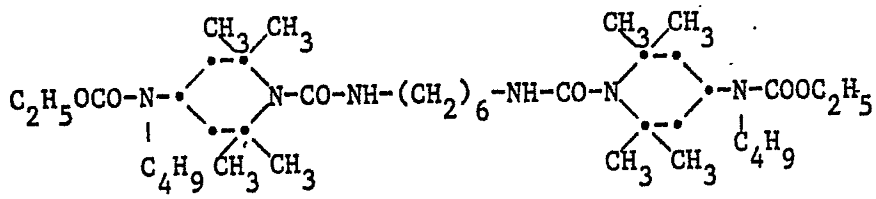

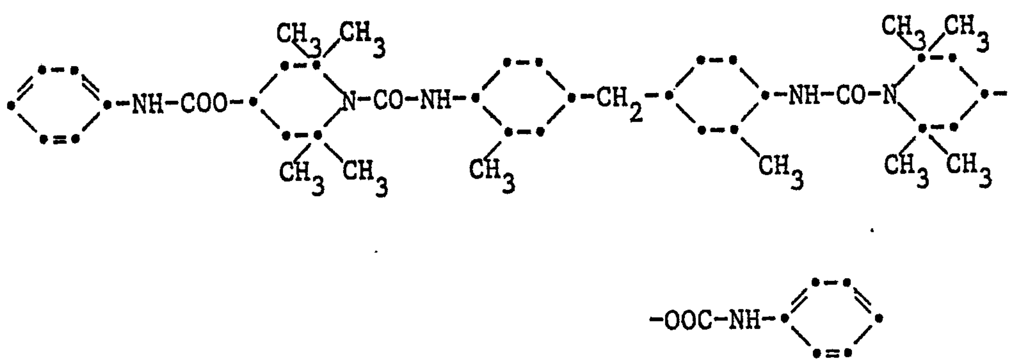

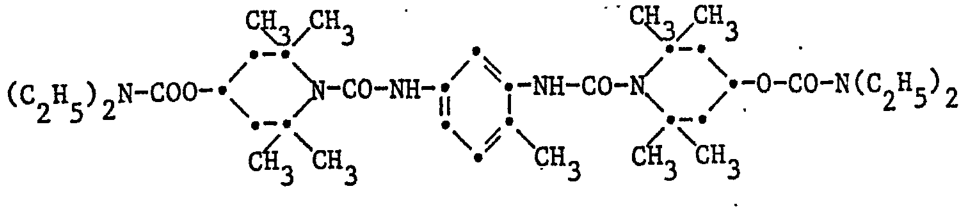

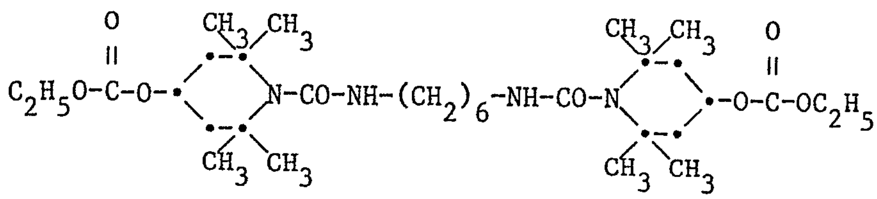

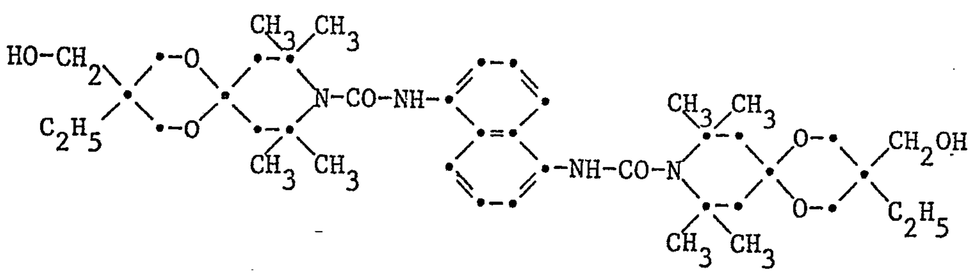

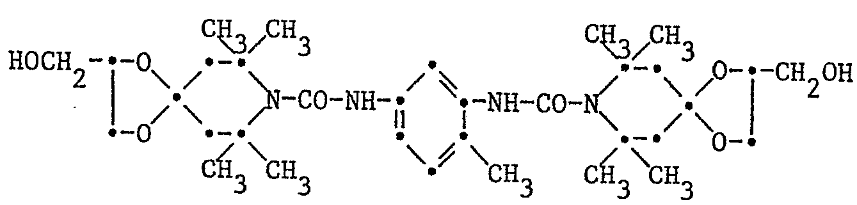

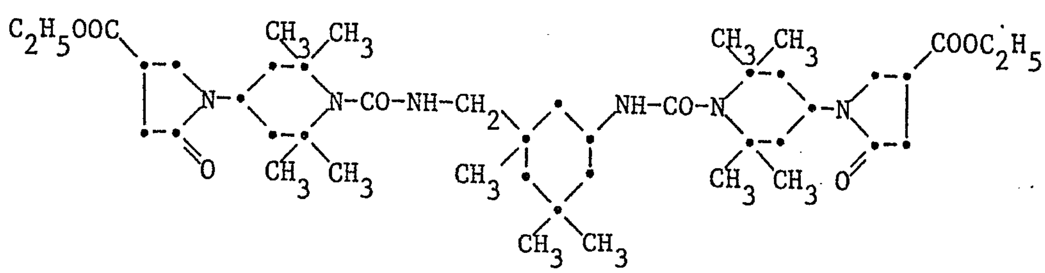

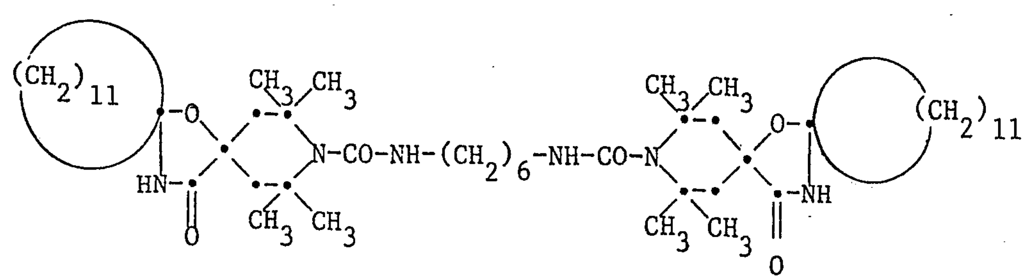

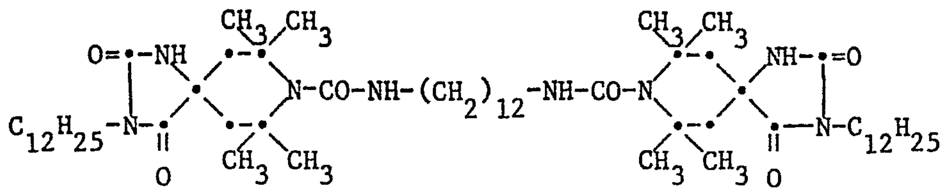

Wie eingangs erwähnt lassen sich die Verbindungen der Formel I in hoher Ausbeute und hoher Reinheit durch Carbamoylierung der entsprechenden in 1-Stellung unsubstituierten Polyalkylpiperidine bei relativ niedriger Temperatur herstellen. Die Erfindung betrifft daher auch ein Verfahren zur Herstellung von Verbindungen der Formel I durch Umsetzung von Verbindungen der Formel VII

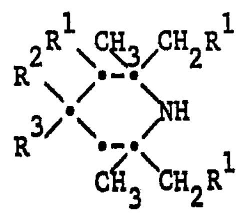

VII

mit Di- oder Triisocyanaten der Formel VIII

$$R^4(NCO)_n \qquad VIII$$

im Molverhältnis von n Molen VII zu 1 Mol VIII in einem inerten Lösungsmittel, dadurch gekennzeichnet, dass man die Reaktion bei -20°C bis +50°C, vorzugsweise bei -5°C bis +30°C, ausführt.

Als inerte Lösungsmittel lassen sich insbesondere Kohlenwasserstoffe, wie Pentan, Hexan, Heptan, Ligroin, Benzin, Cyclohexan, Benzol, Toluol oder Xylol, und Ether, wie z.B. Diethyl- oder Diisopropylether, Tetrahydrofuran, Dioxan oder 1,2-Dimethoxyethan, verwenden.

Vorteilhafterweise legt man eine Lösung der Verbindung VII vor und gibt eine Lösung der Verbindung VIII unter Rühren und Kühlung zu. Die Temperatur des Reaktionsgemisches soll dabei unterhalb 50°C, vorzugsweise unterhalb 30° gehalten werden.

Auf ein Mol Diisocyanat verwendet man 2 Mol der Verbindung VII, auf ein Mol Triisocyanat verwendet man 3 Mol der Verbindung VII. Die Reaktion kann durch Zusatz von katalytischen Mengen von in organischen Lösungsmitteln löslichen Basen beschleunigt werden. Beispiele hierfür sind 1,4-Diazabicyclo[2.2.2]octan oder 1,8-Diazabicyclo[5.4.0]undec-7-en. Ein solcher Katalysator ist jedoch nicht notwendig.

Aus der Reaktionslösung fallen die gebildeten Produkte direkt kristallin aus oder können durch Kühlung zur Kristallisation gebracht werden oder man destilliert zuerst einen Teil oder alles Lösungsmittel ab und lässt dann das Produkt in der Kälte kristallisieren. Man erhält so die Produkte in hoher Reinheit. Auch wenn die Möglichkeit zur Bildung von Nebenprodukten besteht, entsteht bei niedrigen Temperaturen die gewünschte 1-Carbamoylverbindung in hoher Ausbeute, was die überraschende Selekivität des Verfahrens beweist. Beispielsweise entstehen bei der Umsetzung von Diisocyanaten mit 2 Mol des 4-Hydroxy-2,2,6,6-tetramethylpiperidin gemäss dem vorliegenden Verfahren bei Raumtemperatur (20-25°C) in hoher Ausbeute die 1-Carbamoylverbindungen

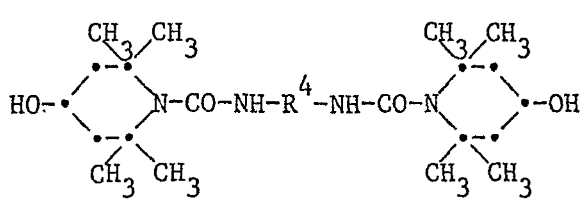

Während bei der bekannten Ausführung in der Wärme (Rückfluss Toluol) vorwiegend die 4-Carbamoyloxyverbindungen

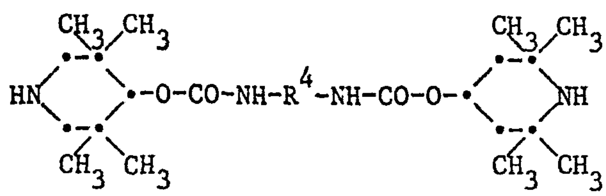

entstehen.

Die Verbindungen der Formel I können als Stabilisatoren für lichtempfindliche organische Materialien verwendet werden, wie z.B. für Kosmetika oder farbphotographische Schichten, insbesondere aber für organische Polymere. Neben einer ausgeprägten Lichtschutz-Wirkung haben die Verbindungen der Formel I auch eine gewisse stabilisierende Wirkung gegen thermische und oxidative Alterung des Polymeren. Beispiele für solche Polymere sind die folgenden:

1. Polymere von Mono- und Diolefinen, beispielsweise Polyethylen (das gegebenenfalls vernetzt sein kann), Polypropylen, Polyisobutylen, Polybuten-1, Polymethylpenten-1, Polyisopren oder Polybutadien sowie Polymerisate von Cycloolefinen, wie z.B. von Cyclopenten oder Norbornen.

2. Mischungen der unter 1) genannten Polymeren, z.B. Mischungen von Polypropylen mit Polyäthylen oder mit Polyisobutylen.

3. Copolymere von Mono- und Diolefinen untereinander oder mit anderen Vinylmonomeren, wie z.B. Ethylen-Propylen-Copolymere, Propylen-Buten-1-Copolymere, Propylen-Isobutylen-Copolymere, Ethylen-Buten-1-Copolymere, Propylen-Butadien-Copolymere, Isobutylen-Isopren-Copolymere, Ethylen-Alkylacrylat-Copolymere, Ethylen-Alkylmethacrylat-Copolymere, Ethylen-Vinylacetat-Copolymere oder Ethylen-Acrylsäure-Copolymere und deren Salze (Ionomere), sowie Terpolymere von Ethylen mit Propylen und einem Dien, wie Hexadien, Dicyclopentadien oder Ethylidennorbornen.

4. Polystyrol.

5. Copolymere von Styrol oder α-Methylstyrol mit Dienen oder Acrylderivaten, wie z.B. Styrol-Butadien, Styrol-Acrylnitril, Styrol-Ethylmethacrylat, Styrol-Butadien-Ethylacrylat, Styrol-Acrylnitril-Methylacrylat; Mischungen von hoher Schlagzähigkeit aus Styrol-Copolymeren und einem anderen Polymer, wie z.B. einem Polyacrylat, einem Dien-Polymeren oder einem Ethylen-Propylen-Dien-Terpolymeren; sowie Block-Copolymere des Styrols, wie z.B. Styrol-Butadien-Styrol, Styrol-Isopren-Styrol, Styrol-Ethylen/Butylen-Styrol oder Styrol-Ethylen/Propylen-Styrol.

6. Pfropfcopolymere von Styrol, wie z.B. Styrol auf Polybutadien, Styrol und Acrylnitril auf Polybutadien, Styrol und Maleinsäureanhydrid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 5) genannten Copolymeren, wie z.B. als sogenannte ABS, MBS, ASA oder AES-Polymere bekannt sind.

7. Halogenhaltige Polymere, wie z.B. Polychloropren, Chlorkautschuk, chloriertes oder chlorsulfoniertes Polyethylen, Epichlorhydrinhomo- und -copolymere, insbesondere Polymere aus halogenhaltigen Vinylverbindungen, wie z.B. Polyvinylchlorid, Polyvinylidenchlorid, Polyvinylfluorid, Polyvinylidenfluorid; sowie deren Copolymere, wie Vinylchlorid—Vinylidenchlorid, Vinylchlorid-Vinylacetat oder Vinylidenchlorid-Vinylacetat.

8. Polymere, die sich von α,β-ungesättigten Säuren und deren Derivaten ableiten, wie Polyacrylate und Polymethacrylate, Polyacrylamide und Polyacrylnitrile.

9. Copolymere der unter 8) genannten Monomeren untereinander oder mit anderen ungesättigten Monomeren, wie z.B. Acrylnitril-Butadien-Copolymere, Acrylnitril-Alkylacrylat-Copolymere, Acrylnitril-Alkoxyalkylacrylat-Copolymere, Acrylnitril-Vinylhalogenid-Copolymere oder Acrylnitril-Alkylmethacrylat-Butadien-Terpolymere.

10. Polymere, die sich von ungesättigten Alkoholen und Aminen bzw. deren Acylderivaten oder Acetalen ableiten, wie Polyvinylalkohol, Polyvinylacetat-, -stearat, -benzoat, -maleat, Polyvinylbutyral, Polyallylphthalat, Polyallylmelamin.

11. Homo- und Copolymere von cyclischen Ethern, wie Polyalkylenglykole, Polyethylenoxyd, Polypropylenoxyd oder deren Copolymere mit Bisglycidylethern.

12. Polyacetale, wie Polyoxymethylen, sowie solche Polyoxymethylene, die Comonomere, wie z.B. Ethylenoxyd enthalten.

13. Polyphenylenoxyde und -sulfide.

14. Polyurethane, die sich Polyestern und Polybutadienen mit endständigen Hydroxylgruppen einerseits und aliphatischen oder aromatischen Polyisocyanaten andererseits ableiten, sowie deren Vorprodukte (Polyisocyanate, Polyole, Präpolymere).

15. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, Polyamid 6/10, Polyamid 11, Polyamid 12, Poly-2,4,4-trimethylhexamethylen-terephthalamid, Poly-m-phenylen-isophthalamid, sowie deren Copolymere mit Polyethern, wie z.B. mit Polyethylenglykol, Polypropylenglykol oder Polytetramethylenglykol.

16. Polyharnstoffe, Polyimide und Polyamid-imide.

17. Polyester, die sich von Dicarbonsäuren und Diolen und/oder von Hydroxycarbonsäuren oder den entsprechenden Lactonen ableiten, wie Polyethylenterphthalat, Polybutylenterphthalat, Poly-1,4-dimethylol-cyclohexanterephthalat, Poly-[2,2-bis(4-hydroxyphenyl)-propan]-terephthalat, Polyhydroxybenzoate, sowie Block-Polyether-ester, die sich von Polyethern mit Hydroxyendgruppen, Dialkoholen und Dicarbonsäuren ableiten.

18. Polycarbonate.

19. Polysulfone und Polyethersulfone.

20. Vernetzte Polymere, die sich von Aldehyden einerseits und Phenolen, Harnstoff und Melamin andererseits ableiten, wie Phenol-Formaldehyd-, Harnstoff-Formaldehyd- und Melamin-Formaldehydharze.

21. Trocknende und nicht-trocknende Alkydharze.

22. Ungesättigte Polyesterharze, die sich von Copolyestern gesättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikation.

23. Vernetzbare Acrylharze, die sich von substituierten Acrylsäureestern ableiten, wie z.B. von Epoxyacrylaten, Urethan-acrylaten oder Polyesteracrylaten.

24. Alkydharze, Polyesterharze und Acrylatharze, die mit Melaminharzen, Harnstoffharzen, Polyisocyanaten oder Epoxidharzen vernetzt sind.

25. Vernetzte Epoxidharze, die sich von Polyepoxiden ableiten,

26. Natürliche Polymere, wie Cellulose, Naturkautschuk, Gelatine, sowie deren polymeranalog chemisch abgewandelten Derivate, wie Celluloseacetate, -propionate und -butyrate, bzw. die Celluloseether, wie Methylcellulose.

Von besonderer Bedeutung ist die Stabilisierung von Lackharzen wie Alkyd-, Acryl- und Polyesterharzen, insbesondere in ihrer Verwendung für säurekatalysierte Einbrennlacke.

Die erfindungsgemässen Stabilisatoren werden den Polymeren in einer Konzentration von 0,01 bis 4 Gew.-%, berechnet auf das zu stabilisierende Material, zugesetzt. Vorzugsweise werden 0,1 bis 2 Gew.-% der Verbindungen, berechnet auf das zu stabilisierende Material, in dieses eingearbeitet.

Die Einarbeitung kann vor, während oder nach der Polymeriation erfolgen, beispielsweise durch Einmischen der Verbindungen und gegebenenfalls weiterer Additive in die Schmelze nach den in der Technik üblichen Methoden, vor oder während der Formgebung. Bei Lacken erfolgt der Zusatz vorzugsweise zur Lösung des Lackes vor dessen Applikation.

Die Stabilisatoren können auch in Form eines Masterbatches, der diese Verbindungen beispielsweise in einer Konzentration von 2,5 bis 25 Gew.-% enthält, den zu stabilisierenden Polymeren zugesetzt werden.

Ausser den Verbindungen der Formel I können den Polymeren auch noch andere, bekannte Stabilisatoren zugesetzt werden. Dies können z.B. Antioxydantien, Lichtschutzmittel oder Metalldesaktivatoren sein, oder auch Costabilisatoren wie z.B. solche vom Typ der Phosphorigsäureester. Weiterhin können sonstige in der Kunststofftechnologie übliche Zusätze wie z.B. Flammschutzmittel, Antistatika, Weichmacher, Gleitmittel, Treibmittel, Pigmente, Verstärkungsstoffe oder Füllstoffe zugesetzt werden.

Bei der Mitverwendung bekannter Stabilisatoren können synergistische Effekte auftreten, was besonders bei Mitverwendung von anderen Lichtschutzmitteln oder von organischen Phosphiten häufig der Fall ist. Von besonderer Bedeutung ist die Mitverwendung von Antioxydantien bei der Stabilisierung von Polyolefinen.

Die Erfindung betrifft daher auch die durch Zusatz von 0,1 bis 4 Gew.-% einer Verbindung der Formel I stabilisierten organischen Polymeren, die gegebenenfalls noch andere bekannte und übliche Zusätze enthalten können. Die so stabilisierten Kunststoffe können in verschiedenster Form angewendet werden, z.B. als Folien, Fasern, Bändchen, Profile, insbesondere aber als Bindemittel für Lacke.

Die Herstellung und Verwendung der erfindungsgemässen Verbindungen wird in den folgenden Beispielen näher beschrieben. Teile bedeuten darin Gewichtsteile und % Gewichtsprozente. Die Temperaturen sind in Celsius-Graden angegeben.

Die Formel

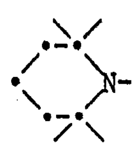

bedeutet darin einen 2,2,6,6-Tetramethylpiperidinrest.

<u>Beispiel 1:</u>

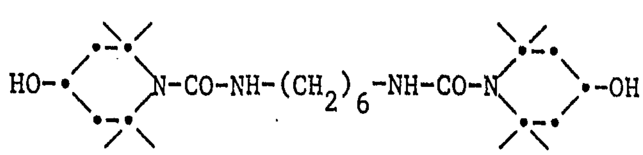

Zu einer Lösung von 31,4 g (0,2 Mol) 4-Hydroxy-2,2,6,6-tetramethylpiperidin in 300 ml wasserfreiem Tetrahydrofuran tropft man bei 22 - 25° unter Rühren innert 7 Stunden regelmässig die Lösung von 16,8 g (0,1 Mol) Hexamethylendiisocyanat in 50 ml Tetrahydrofuran. Anschliessend wird über Nacht bei Raumtemperatur weiter gerührt, wobei eine dicke, weisse Suspension des ausgefallenen Reaktionsproduktes entsteht. Das Reaktionsprodukt wird abfiltriert, mit kaltem Tetrahydrofuran gut nachgewaschen, scharf abgesaugt und im Hochvakuum bei Raumtemperatur getrocknet. Die so erhaltene Dicarbamoylverbindung ist analysenrein und besitzt einen Smp. von 145 - 146°.

Das $^{1}H$-NMR-Spektrum ($CDCl_3$) ist mit der angegebenen Struktur gut verträglich.

| | | | | |
|---|---|---|---|---|
| $C_2H_{50}N_4O_4$ | Ber.: C 64,69 | H 10,44 | N 11,61 % | |
| (482,7) | Gef.: C 64,5 | H 10,6 | N 11,8 %. | |

Analog werden aus einem Polyalkylpiperidin und einem Diisocyanat die in Tabelle 1 aufgeführten Verbindungen hergestellt:

Tabelle 1

| Verbindung Nr. | Formel | Smp. |
|---|---|---|
| 1 | 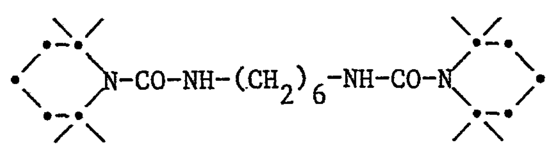 | 132-133° |
| 2 | 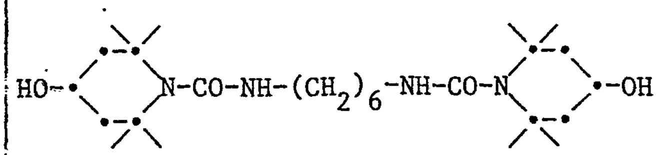 | 145-146° |
| 3 | 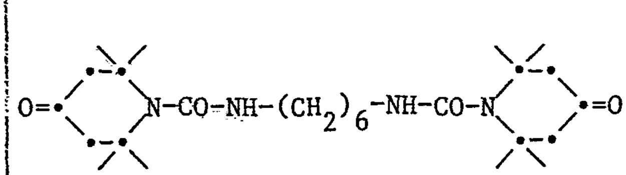 | 124-125,5° |

Tabelle 1 (Fortsetzung)

| Verbindung Nr. | Formel | Smp. |
|---|---|---|
| 4 | 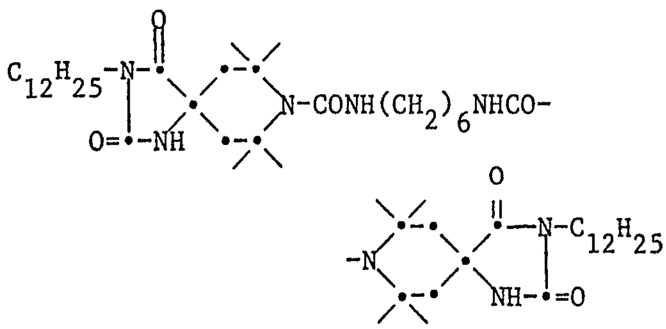 | 119-120° |
| 5 | 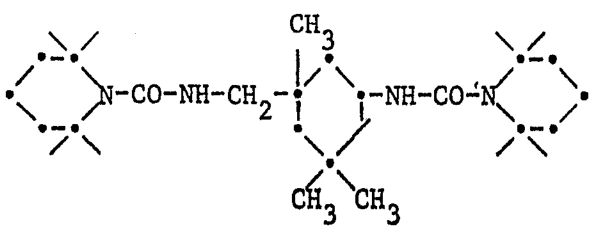 | 120,5-121° |
| 6 | 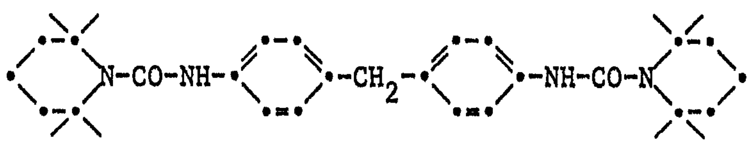 | 124,5-126° |
| 7 | 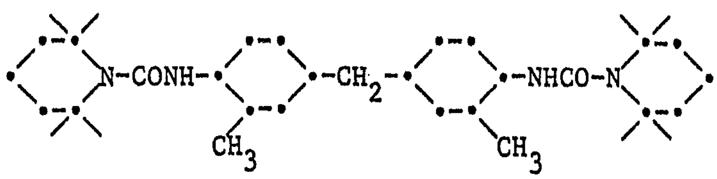 | 110-114° |
| 8 | 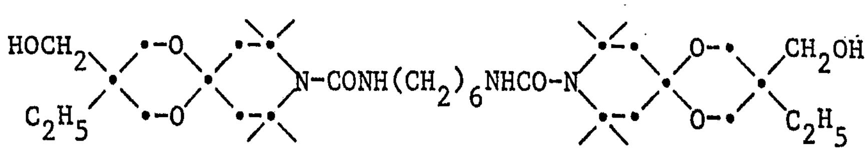 | 113-115° |

Tabelle 1 (Fortsetzung)

| Verbindung Nr. | Formel | Smp. |
|---|---|---|
| 9 | 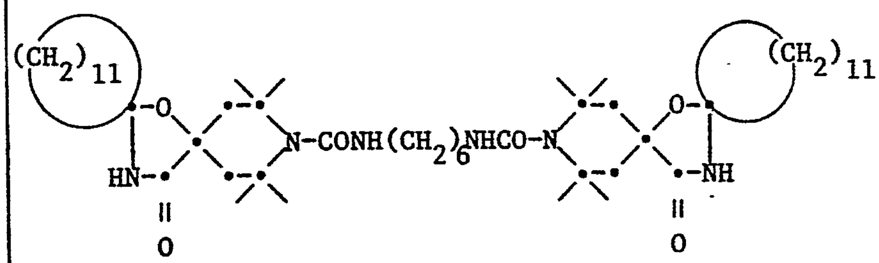 | 172-175° |
| 10 | 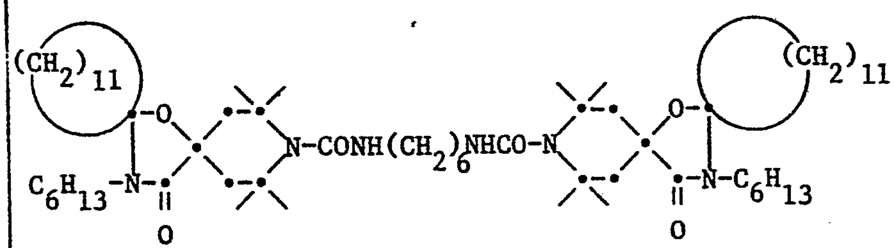 | 128-131° |
| 11 | 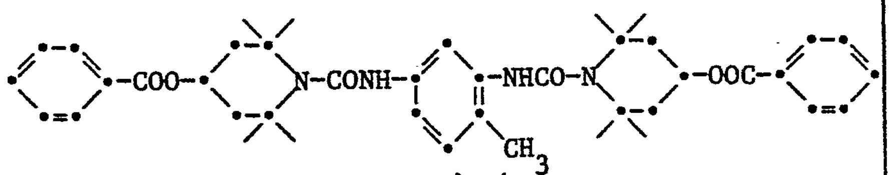 | 95-98° |
| 12 | 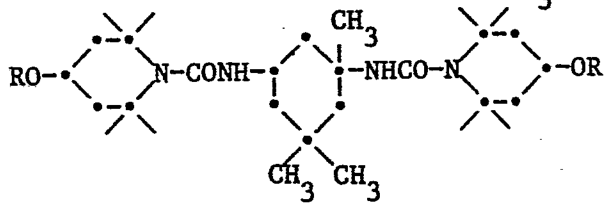 R = 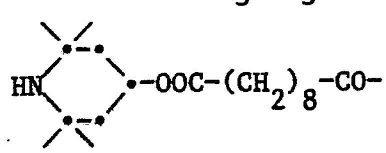 | ~86° (amorph) |
| 13 | 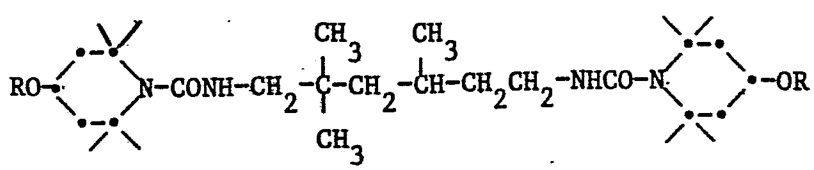 | ~60° (amorph) |

Beispiel 2

100 Teile Polypropylenpulver (Moplen, Fibre grade, der Firme Montedison) werden mit 0,2 Teilen β-(3,5-Di-tert.-butyl-4-hydroxyphenyl)-propionsäure-octadecylester, 0,1 Teilen Calciumstearat und 0,25 Teilen eines Stabilisators der folgenden Tabelle 1 im Brabender-Plastographen bei 200° während 10 Minuten homogenisiert. Die so erhaltene Masse wird möglichst rasch dem Kneter entnommen und in einer Kniehebelpresse zu einer 2-3 mm dicken Platte gepresst. Ein Teil des erhaltenen Rohpresslings wird ausgeschnitten und zwischen zwei Hochglanz-Hartaluminiumfolien mit einer hydraulischen Laborpresse während 6 Minuten bei 260° zu einer 0,1 mm dicken Folie gepresst, die unverzüglich in kaltem Wasser abgeschreckt wird. Aus dieser werden nun Abschnitte ausgestanzt und im Xenotest 1200 belichtet. In regelmässigen Zeitabständen werden diese Prüflinge aus dem Belichtungsapparat entnommen und in einem IR-Spektrophotometer auf ihren Carbonylgehalt geprüft. Die Zunahme der Carbonylextinktion bei 5,85 µm während der Belichtung ist ein Mass für den photooxidativen Abbau des Polymeren (s.L. Balaban et al., J. Polymer Sci, Part C; 22 (1969), 1059 - 1071) und ist erfahrungsgemäss mit einem Abfall der mechanischen Eigenschaften des Polymeren verbunden. Als Mass der Schutzwirkung gilt die Zeit bis Erreichen einer Carbonylextinktion von ca. 0,3, bei welcher die Vergleichsfolie brüchig ist.

| Stabilisator | Belichtungszeit in Stunden |
|---|---|
| ohne | 900 |
| Verbindung Nr. 8 | > 3420 |
| Verbindung Nr. 10 | > 3420 |
| Verbindung Nr. 11 | 2300 |

Patentansprüche

1. Verbindungen der Formel I,

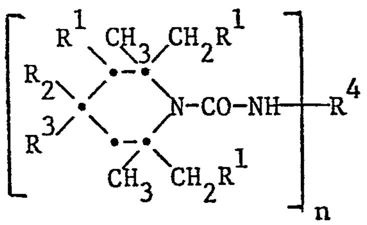 I

worin n 2 oder 3 ist,

$R^1$ Wasserstoff oder $C_1$-$C_4$-Alkyl ist,

$R^2$ Wasserstoff, -OH, $-OR^5$, $-COOR^6$, $-CH_2COOR^6$, $-CH_2CN$, -CN, $-N(R^7)_2$, $-CO-N(R^{7a})_2$, $-CH_2CH_2N(R^7)_2$, $-N(R^{7a})-CO-R^8$, $-O-CO-R^9$, $-OCH_2CH_2-CN$, $-N(R^{7a})-CO-O-R^{10}$, $-O-CO-O-R^{10}$, $-O-CO-N(R^{7a})_2$ oder eine Gruppe

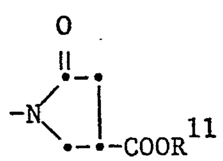 , 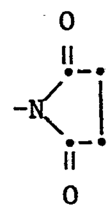 , 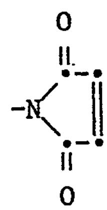 , 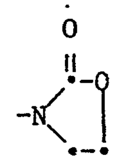

oder 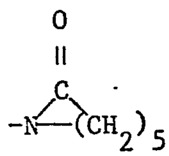 bedeutet,

$R^3$ Wasserstoff ist oder $R^2$ und $R^3$ zusammen O=, NC-CH= oder eine Gruppe der Formel II, III, IV, V oder VI bedeuten,

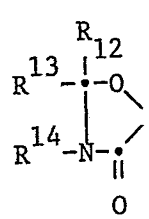 , 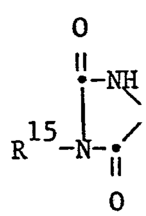 , 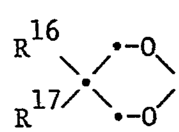 ,

II III IV

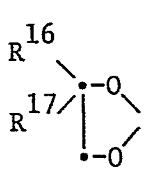 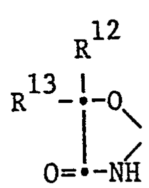

V VI

$R^4$ ein n-wertiger Rest eines aliphatischen, cycloaliphatischen, aromatischen oder aromatisch-aliphatischen Di- oder Triisocyanates ist,

$R^5$ $C_1$-$C_{12}$-Alkyl, $C_3$-$C_{14}$-Oxaalkyl, $C_3$-$C_5$-Alkenyl, Propargyl, Benzyl, oder $-(CH_2CH_2O)_p-H$ mit p = 1-10 bedeutet,

$R^6$ Wasserstoff, $C_1$-$C_{18}$-Alkyl, $C_3$-$C_5$-Alkenyl, $C_5$-$C_8$-Cycloalkyl, $C_6$-$C_{10}$-Aryl, $C_7$-$C_{12}$-Alkylaryl, $C_7$-$C_{12}$-Phenylalkyl oder eine Gruppe der Formel VI bedeutet,

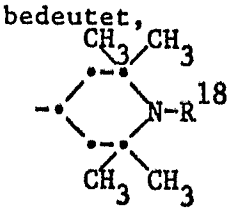

VI

$R^7$ $C_1$-$C_{18}$-Alkyl, $C_2$-$C_{14}$-Oxaalkyl, $C_3$-$C_5$-Alkenyl, $C_5$-$C_8$-Cycloalkyl, $C_7$-$C_{12}$-Phenylalkyl, $C_6$-$C_{10}$-Aryl, $C_2$-$C_4$-Hydroxyalkyl oder $C_7$-$C_{10}$-Cycloalkyl-alkyl bedeutet, oder beide Reste $R^7$ zusammen mit dem N-Atom, an das sie gebunden sind, einen 5-7-gliedrigen Ring bilden,

$R^{7a}$ Wasserstoff ist oder eine der für $R^7$ gegebenen Bedeutungen hat,

$R^8$ $C_1$-$C_{12}$-Alkyl, $C_3$-$C_{14}$-Oxaalkyl, durch -COOH oder -COO($C_1$-$C_4$-Alkyl) substituiertes $C_2$-$C_{14}$-Alkyl, $C_2$-$C_5$-Alkenyl, $C_5$-$C_8$-Cycloalkyl, $C_7$-$C_{12}$-Phenylalkyl, $C_6$-$C_{10}$-Aryl oder $C_7$-$C_{12}$-Alkylaryl bedeutet,

$R^9$ $C_1$-$C_{18}$-Alkyl, $C_3$-$C_{14}$-Oxaalkyl, durch -COOH oder -COO($C_1$-$C_4$-Alkyl) substituiertes $C_2$-$C_{14}$-Alkyl, $C_2$-$C_5$-Alkenyl, $C_5$-$C_8$-Cycloalkyl, $C_6$-$C_{10}$-Aryl, $C_7$-$C_{12}$-Phenylalkyl, $C_7$-$C_{12}$-Alkylaryl oder durch OH und 1 - 3 $C_1$-$C_4$-Alkylgruppen substituiertes Phenyl oder $C_7$-$C_{10}$-Phenylalkyl bedeutet,

$R^{10}$ $C_1$-$C_{12}$-Alkyl, $C_3$-$C_5$-Alkenyl, $C_5$-$C_8$-Cycloalkyl, Phenyl oder $C_7$-$C_{12}$-Phenylalkyl bedeutet,

$R^{11}$ Wasserstoff oder $C_1$-$C_8$-Alkyl ist,

$R^{12}$ Wasserstoff, $C_1$-$C_{12}$-Alkyl, $C_5$-$C_8$-Cycloalkyl oder Benzyl und

$R^{13}$ $C_1$-$C_{12}$-Alkyl, $C_5$-$C_8$-Cycloalkyl oder Phenyl ist oder

$R^{12}$ und $R^{13}$ zusammen mit dem C-Atom, an das sie gebunden sind, einen $C_6$-$C_{12}$-Cycloalkan- oder Alkylcycloalkanring bilden,

$R^{14}$ Wasserstoff, $C_1$-$C_{12}$-Alkyl, $C_3$-$C_5$-Alkenyl, $C_5$-$C_8$-Cycloalkyl, $C_2$-$C_4$-Hydroxyalkyl, $C_2$-$C_{10}$-Alkanoyl, Benzoyl, $C_7$-$C_{12}$-Phenylalkyl oder eine Gruppe R-NH-CO- ist, worin R $C_1$-$C_8$-Alkyl oder Phenyl bedeutet,

$R^{15}$ Wasserstoff, $C_1$-$C_{18}$-Alkyl, $C_3$-$C_5$-Alkenyl, $C_2$-$C_4$-Hydroxyalkyl, $C_5$-$C_8$-Cycloalkyl, $C_7$-$C_{12}$-Phenylalkyl oder Glycidyl bedeutet,

$R^{16}$ Wasserstoff oder $C_1$-$C_4$-Alkyl ist,

$R^{17}$ Wasserstoff, Methyl, Ethyl oder Hydroxymethyl bedeutet und

$R^{18}$ Wasserstoff, $C_1$-$C_4$-Alkyl, Allyl, Benzyl, Acetyl oder Acryloyl bedeutet.

2. Verbindungen der Formel I gemäss Anspruch 1, worin n 2 ist und $R^4$ ein zweiwertiger gesättigter aliphatischer Rest mit 2-16 C-Atomen, ein zweiwertiger cycloaliphatischer Rest mit 5-20 C-Atomen oder ein zweiwertiger aromatischer oder aromatisch-aliphatischer Rest mit 6 - 20 C-Atomen ist, oder n 3 ist und $R^4$ ein dreiwertiger aromatisch-aliphatischer Rest mit 10-30 C-Atomen ist.

3. Verbindungen der Formel I gemäss Anspruch 1, worin $R^1$ Wasserstoff ist.

4. Verbindungen der Formel I gemäss Anspruch 1, worin $R^1$ Wasserstoff, $R^2$ -OH, $-OR^5$, $-CH_2COOR^6$, $-CH_2CN$, $-N(R^7)-CO-R^8$, $-O-CO-R^9$, $-N(R^7)-CO-OR^{10}$ oder $-O-CO-N(R^7)_2$ bedeutet, $R^3$ Wasserstoff ist oder $R^2$ und $R^3$ zusammen O= oder eine Gruppe der Formeln II, III, IV oder V bedeuten, $R^4$ bei n = 2 $C_2$-$C_{12}$-Alkylen, Phenylen, Naphthylen, Tolylen oder eine Gruppe der Formeln

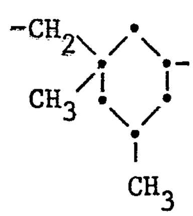 , 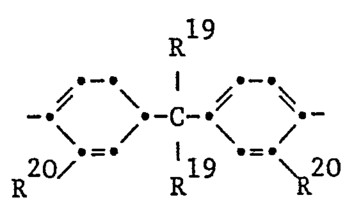 oder

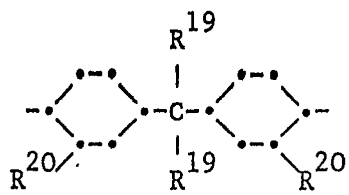

bedeutet, worin $R^{19}$ Wasserstoff, Methyl oder Ethyl ist und $R^{20}$ Wasserstoff oder Methyl ist,
und bei n = 3 eine Gruppe der Formel

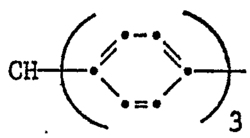 oder 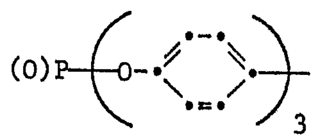 bedeutet,

$R^5$ $C_1$-$C_{12}$-Alkyl, $C_3$-$C_5$-Alkenyl oder Benzyl ist, $R^6$ $C_1$-$C_4$-Alkyl oder Cyclohexyl ist, $R^7$ $C_1$-$C_{12}$-Alkyl, Cyclohexyl, Allyl oder Phenyl ist, $R^8$ und $R^9$ $C_1$-$C_{12}$-Alkyl, $C_2$-$C_3$-Alkenyl, Phenyl, Benzyl oder Cyclohexyl bedeuten und $R^{10}$ $C_1$-$C_{12}$-Alkyl oder Phenyl ist.

5. Verfahren zur Herstellung von Verbindungen der Formel I gemäss Anspruch 1 durch Umsetzung von Verbindungen der Formel VII

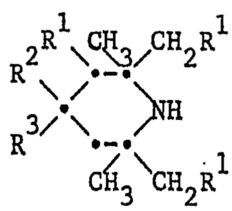 VII

mit Di- oder Triisocyanaten der Formel VIII

$$R^4(NCO)_n \qquad \text{VIII}$$

im Molverhältnis von n Molen VII zu 1 Mol VIII in einem inerten Lösungsmittel, dadurch gekennzeichnet, dass man die Reaktion bei -20°C bis +50°C, vorzugsweise bei -5°C bis +30°C, ausführt.

6. Verwendung von Verbindungen der Formel I gemäss Anspruch 1 als Stabilisator für lichtempfindliche organische Materialien.

7. Verwendung gemäss Anspruch 6, als Stabilisator für organische Polymere.

8. Organische Polymere, enthaltend als Stabilisator 0,01 bis 4 Gew.-% mindestens einer Verbindung der Formel I gemäss Anspruch 1.

9. Organische Polymere gemäss Anspruch 8 als Bindemittel für Lacke.

FO 7.3 SA/bg*